# EUROPEAN PATENT APPLICATION

(11) **EP 4 674 413 A1**
(43) Date of publication of application: **07.01.2026**
(21) Application number: 25186773.5
(22) Date of filing: 01.07.2025
(51) Int. Cl.: A61K 31/122, A61K 31/455, A61P 9/00, A61P 9/02

(54) **SYNERGISTIC COMBINATION OF 1-METHYLNICOTINAMIDE (MNA) AND VITAMIN K FOR USE IN THE TREATMENT OR PREVENTION OF CARDIOVASCULAR COMPLICATIONS OF ANTICANCER THERAPIES**

(30) Priority: 01.07.2024 PL 44909224
(71) Applicant: UNIWERSYTET JAGIELLONSKI, 31-007 Kraków (PL)
(72) Inventor: Chlopicki, Stefan, 31-138 Kraków (PL); Bar, Anna, 30-724 Kraków (PL); Marczyk, Brygida, 32-095 Poskwitów (PL)
(74) Representative: Witek, Rafal

(57) **Abstract**

A synergistic combination of agents with a protective effect on vascular endothelial function is disclosed, for use in the treatment and prevention of complications of anticancer therapies.

## Description

The invention relates to a synergistic combination of agents showing protective effects on the vascular endothelium, in particular a combination of 1-methylnicotinamide (MNA) and vitamin K, for use in the treatment or prevention of cardiovascular complications of anticancer therapies. The combination is particularly suitable for the treatment and prevention of chronic anticancer therapy complications, especially in patients with current endothelial dysfunction caused by a factor other than the anticancer therapy. In particular, the invention covers the prevention of adverse effects of tyrosine kinase inhibitor (TKI)-based therapies, which are associated with adverse cardiovascular effects.

TKIs have revolutionised the treatment of chronic myeloid leukaemia (CML) and are now also used to treat many other cancers, but the use of these inhibitors is associated with an increased risk of adverse cardiovascular events (Douxfils *et al.,* 2016). Adverse effects of anticancer therapies on the blood vessel wall are now a well-known complication of anticancer therapy and result in the development of hypertension, thrombotic complications or other cardiovascular episodes including the development of heart failure (Iurlo *et al.,* 2023). The occurrence of cardiovascular-related adverse effects in patients treated with TKIs may limit the long-term use of these drugs and negatively affect patient survival. For example, in CML patients treated with nilotinib, adverse cardiovascular events occur in10 per cent of patients after five years of treatment and 25 per cent of patients after 10 years of treatment, in the ENESTnd study (Kantarjian *et al.,* 2021). Meanwhile, in the PACE study, arterial occlusive events (AOEs) occurred in 31% of patients treated for 5 years with ponatinib (Cortes *et al.,* 2018).

Many studies have used successive generations of anticancer drugs from the TKI group and explored the mechanisms of their vasculotoxic effects (Loren *et al.,* 2015; Herrmann *et al.,* 2016; Schmaier *et al*., 2016; Gover-Proaktor *et al.,* 2017, 2019; Hadzijusufovic *et al.,* 2017; Haguet *et al.,* 2020; Lin *et al.,* 2023), yet no solutions have been found to counteract the vascular toxicity of potent TKI anticancer drugs, while ensuring high antitumour efficacy of the drug (Hochhaus *et al.,* 2016). This is important because cancer treatment requires long-term use of TKI-based therapy. It is therefore necessary to develop a way to mitigate or eliminate the side effects of the drugs used (Kantarjian *et al.,* 2021) based on agents that are safe and non-toxic.

Recent experimental studies suggest a number of potential mechanisms that may be involved in the occurrence of cardiovascular complications of anticancer therapies. In particular, TKIs have adverse effects on vascular endothelial function (Pasvolsky *et al.,* 2015 Loren *et al.,* 2015; Herrmann *et al.,* 2016; Schmaier *et al.,* 2016; Gover-Proaktor *et al.,* 2017, 2019; Hadzijusufovic *et al.,* 2017; Haguet *et al.,* 2020; Lin *et al.,* 2023) and this adverse effect of these drugs on endothelial function may lead to cardiovascular complications in patients receiving TKI (Cortes *et al.,* 2018) (Kantarjian *et al.,* 2021).

Furthermore, endothelial dysfunction is often present in oncology patients even before they start anticancer treatment, due to the developing cancer, co-morbidities such as cardiovascular disease or older age, which may exacerbate the adverse effect of TKI-based treatment, as well as further increase the risk of cardiovascular events. In such patients, vascular endothelial function is impaired at baseline, and the protection from the vasotoxic effects of anticancer therapy is particularly important. As demonstrated in experimental studies, impaired vascular endothelial function can be improved by 1-methylnicotinamide (MNA) (Bar *et al.,* 2017). In the cited study, a 2-month MNA-based therapy (100 mg/kg/day) improved impaired endothelial function in mice with developing atherosclerosis. The therapeutic activity of MNA has also been demonstrated in several papers describing the use of the compound in ameliorating inflammatory response in skin diseases (G 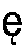 bicki *et al.,* 2003; Woźniacka *et al*., 2005). Furthermore, the anti-thrombotic (Ch 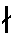 opicki *et al.,* 2007), anti-inflammatory (Bryniarski *et al.,* 2008) and gastroprotective (Brzozowski *et al.,* 2008) effects of MNA have been described. On the other hand, as shown in another paper (Bar *et al.,* 2019), endothelial function impairment in mice with developing atherosclerosis can be improved using vitamin K (K2-MK7) which, at low or high dose (0.05 mg/kg/day - 10 mg/kg/day), exerted similar effects on endothelial function. Improvement in endothelial function can also be induced by vitamin K1 (Kieronska *et al.,* 2024).

The aim of the invention is to provide a solution that would allow the negative effects of anticancer therapies on the vascular endothelium, in particular therapies carried out using TKIs, to be eliminated.

The subject of the invention is the combination and its medical use, defined in detail in the appended claims.

Unexpectedly, it has been found that the use of the combination of MNA and vitamin K leads to the mitigation or even complete elimination of adverse cardiovascular side effects involving impaired endothelial function developing due to therapies using TKIs.

In the studies that led to the present invention, it was found that the use of the claimed combination leads to an unexpected synergy of their action. According to the invention, this synergy is that the used combination of MNA and vitamin K not only completely prevents the negative effects of TKI therapy on the vascular endothelium, but completely unexpectedly reverses the state of impaired endothelial function to a state of completely normal endothelial function as observed in young mice. Such an effect is not produced by any of the components of the combination administered alone.

Unexpectedly, in the experiment that led to the present invention, such an effect was observed in older mice with pre-existing endothelial dysfunction, which were then subjected to TKI therapy further exacerbating the cardiovascular dysfunction.

It is therefore the subject of the invention to use a composition of agents exhibiting protective effects on the vascular endothelium, for the treatment and prevention of complications of chronic anticancer therapies, in particular involving the use of TKIs. Preferably, a composition of MNA and vitamin K is used as the composition of agents having a protective effect on the vascular endothelium. Various vitamins grouped as vitamin K can be used for the composition of compounds having a protective effect on the vascular endothelium.

As used in the context of this invention, the term "vitamin K" should be understood to include any of the vitamins grouped as vitamin K and their pharmaceutically active precursors or metabolites. In particular, it may be a natural 2-methyl-1,4-naphthoquinone derivative, preferably chosen from: vitamin K1 (phylloquinone, PK) or vitamin K2 (menaquinone, MK). In the case of vitamin K2, it can exist in different forms (a group of compounds differing in the number of isoprenoid units in the side chain, from 4 to 13, K2-MK4, K2-MK5, up to K2-MK13). In the embodiment described below, K2-MK-7 was used as the vitamin K2.

Preferably, the composition containing a therapeutically effective amount of vitamin K is intended for use in the prevention of complications of anticancer therapies arising from or associated with vascular endothelial dysfunction, in particular in therapy with inhibitors of the Abl tyrosine kinase activity of the Bcr-abl oncoprotein, in the treatment of cancers expressing the Abl tyrosine kinase of the Bcr-abl oncoprotein, particularly Philadelphia chromosome-positive chronic myeloid leukaemia, but also in the treatment of other cancers treated with TKI drugs.

Preferably, the tyrosine kinase activity inhibitors used in therapy are first-generation inhibitors of the Abl tyrosine kinase activity of the Bcr-abl oncoprotein (imatinib), second-generation 2GTKIs (bosutinib, nilotinib, dasatinib), 3GTKIs (ponatinib and asciminib) or other receptor tyrosine kinase inhibitors such as: erlotinib, gefitinib, afatinib, lapatinib, semaxinib, vatalanib, sunitinib, sorafenib, tandutinib, leflunomide, ibrutinib, acalabrutinib or zanubrutinib.

Preferably, the amount of vitamin K per single dose is between 100 µg and 50 mg.

Complications of the anticancer therapies may include systemic hypertension, pulmonary hypertension, peripheral arterial disease (PAD), deep vein thrombosis (DVT), limb ischemia, thrombotic episodes such as acute myocardial infarction (AMI), cerebrovascular episodes or clinical worsening of atherosclerosis in the coronary, cerebral or peripheral circulation.

Unexpectedly, the use of the proposed combination of compounds with a positive effect on endothelial function, reversed the endothelial function impairment induced by anticancer therapy, especially with TKIs, and, in addition, also allowed for the reversal of endothelial function impairment induced by additional factors, e.g. due to the patient's age or coexisting cardiovascular risk factors. Therefore, the use of compositions to protect against the effects of anticancer therapies may be of particular interest in patients burdened by endothelial dysfunction resulting not only from the adverse effects of TKIs on endothelial function, but also from developing cancer, comorbidities or older age, where the use of single compounds may have an insufficient effect.

In order to better explain the invention, it is presented in embodiments not limiting its scope, described below.

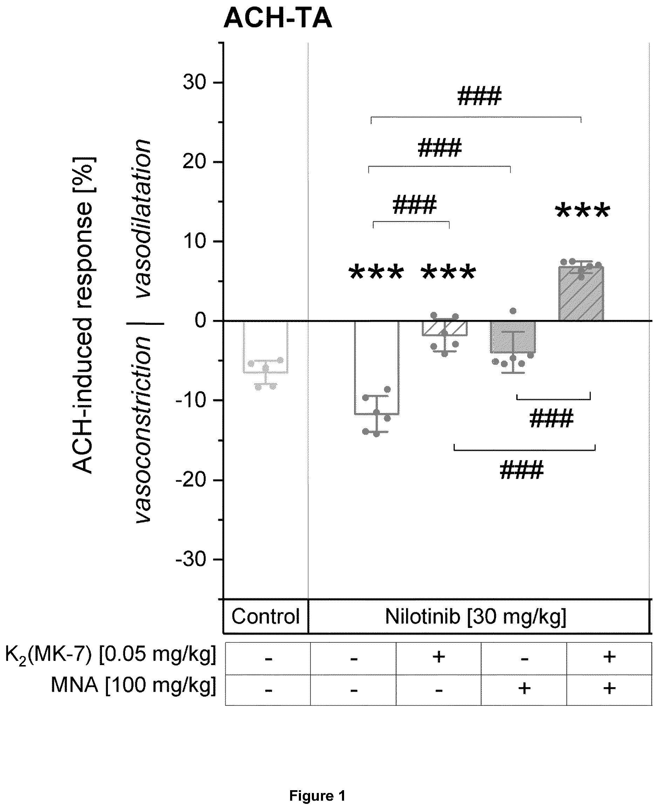

Figure 1 shows the effect of 4-week MNA and vitamin K (K2-MK-7) treatment on vascular endothelial function *in vivo* in nilotinib-treated mice. Endothelium-dependent thoracic aortic response (ACH-TA) 30 minutes after acetylcholine (Ach) *i.p.* administration in 68-74-week-old male C57BL/6 mice after 4-week nilotinib therapy (dose 30 mg/kg b.w./day) compared with - a group of TKI-untreated mice (Control). Concurrent vasoprotective therapy included MNA (dose 100 mg/kg b.w./day) and vitamin K2-MK-7 (dose 0.05 mg/kg b.w./day). Results are presented as mean ± SD. Size of groups: n=5-6. Statistical analysis with designations: one-way analysis of variance with Tukey's post hoc test; ***p<0.001 for nilotinib-treated mice compared with the group of TKI-untreated mice, ###p<0.001 for nilotinib-treated mice compared with mice treated with additional MNA or vitamin K2-MK-7 therapy.

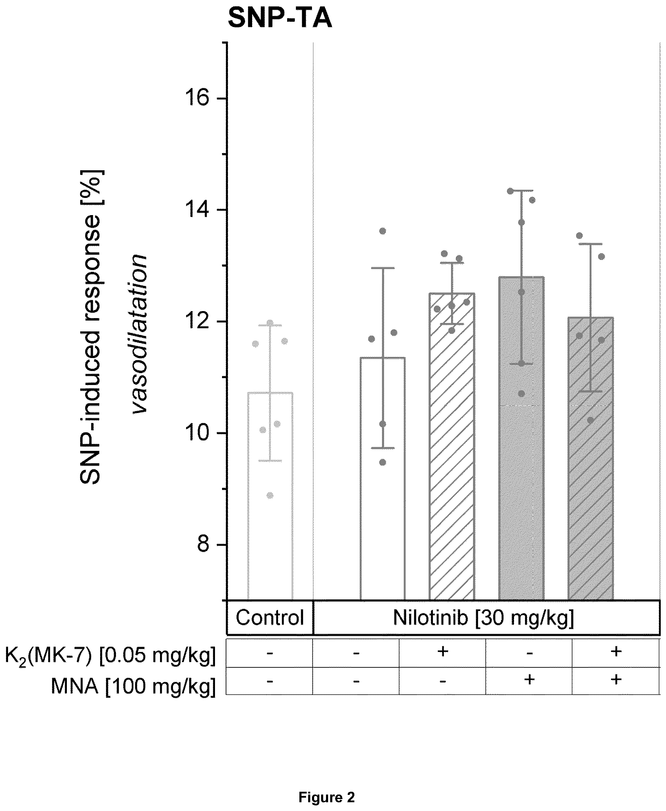

Figure 2 presents the results of an experiment confirming the lack of effect of 4-week nilotinib therapy and vasoprotective therapy with MNA and vitamin K (K2-MK-7) on endothelium-independent vasodilator function. The endothelium-independent thoracic aortic response (SNP-TA) was studied 30 minutes after sodium nitroprusside (SNP) *i.v.* administration in 72-74-week-old male C57BL/6 mice after 4-week nilotinib therapy (dose 30 mg/kg/day) compared with a group of TKI-untreated mice (Control). Concurrent vasoprotective therapy included MNA (dose 100 mg/kg b.w./day) and vitamin K2-MK-7 (dose 0.05 mg/kg b.w./day). Results are presented as mean ± SD. Size of groups: n=5-6. Statistical analysis including designations: one-way analysis of variance with Tukey's post hoc test.

The following describes the results of the evaluation of the effect of the used composition of MNA and vitamin K (K2-MK7) on endothelial dysfunction induced by chronic administration of nilotinib (a second-generation TKI drug) in mice with concomitant age-dependent endothelial dysfunction, which mimics the degree of endothelial dysfunction in cancer patients often burdened with endothelial dysfunction also prior to anticancer therapy. The results of the invention are also shown in the accompanying figures.

### Example 1. Use of MNA and vitamin K composition to prevent negative effects of TKI drugs on endothelial function, in aged mice (68-72 weeks) treated with nilotinib.

Experimental description: Wild-type mice (strain C57/BL6, Centre for Experimental Medicine, Bia 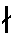 ystok, Poland) aged 64-70 weeks (n=5-6/group) were used in the experiment. The age of mice was selected to provide age-related endothelial dysfunction, mimicking the degree of endothelial dysfunction in older cancer patients. Over a 4-week period, mice were treated with nilotinib (STI Poznań, Poland; CAS: 923288-90-8), which is an inhibitor of the Abl tyrosine kinase activity of the Bcr-abl oncoprotein, and MNA and/or vitamin K (K2-MK7) administered alone or as a combination of both compounds. Both the TKI and the vasoprotective compounds were administered to mice in AIN93G feed (Zoolab, Poland). The mice received the following doses of each compound: 100 mg/kg b.w./day MNA; 0.05 mg/kg b.w./day vitamin K2-MK7; and 30 mg/kg b.w./day nilotinib. The choice of MNA dose was based on previous studies (Ch 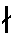 opicki *et al.,* 2007; Mateuszuk *et al.,* 2016) showing that MNA at 100 mg/kg b.w./day has therapeutic properties for endothelial dysfunction caused by hypercholesterolaemia. The dose of 0.05 mg/kg b.w./day of vitamin K2-MK7, on the other hand, was the lowest dose used in previous work (Bar *et al.,* 2017, 2019) that also improved endothelial function in a mouse model of hypercholesterolaemia, already after 2 weeks of use. The nilotinib dose (30 mg/kg/day) was a dose selected to induce vascular endothelial function impairment while being compatible with nilotinib dosing in humans. The dose of nilotinib used in humans is a maximum of 800 mg/day, with 400 mg/day being the most commonly used dose. Thus, in humans, the therapeutic dose is in the range of approximately 6-11 mg/kg (for a 70-kg human), which, given the accelerated metabolism of mice, corresponds to a dose 3-10 times higher. The 30 mg/kg/day used in the experiments conducted in mice therefore represents a dose level compatible with the therapeutic dose used in anticancer therapy.

After the 4-week therapy, *in vivo* studies were performed in mice using magnetic resonance imaging (9.4 T MRI scanner, BioSpec 94/20 USR, Bruker, Germany). The *in vivo* study was conducted in a mouse (lying supine) under inhalation anaesthesia using isoflurane at a concentration of 1.5-1.75% in a 1:2 mixture of oxygen and air, with simultaneous monitoring of the animal's condition using a respiratory sensor, a temperature sensor (maintaining heat at 37°C with circulating warm water) and an ECG system (SA Inc., Stony Brook, USA).

Vascular endothelial function was assessed in mice based on the observation of aortic diameter in diastole (thoracic part of the aorta) 30 minutes after administration of acetylcholine (Ach, Sigma-Aldrich, 50 µl, 16.6 mg/kg, *i.p*.)*.* In a healthy individual, vasodilatation occurs after Ach administration. If vascular endothelial function is impaired, vasodilatation is reduced or even vasoconstriction may occur. In addition, a vascular response test to sodium nitroprusside (SNP, 1 mg/kg *i.v*.) was performed to exclude smooth muscle dysfunction that could contribute to an impaired vascular functional response. The vascular response was studied by comparing two 3D time-resolved images of the vessels before and 30 minutes after Ach administration in aorta. These images were acquired using the IntraGate^{™} FLASH 3D cine sequence and reconstructed using IntraGate 1.2.b.2 macro (Bruker). Vascular volume analysis was performed using ImageJ 1.46r software (NIH Bethesda, USA). Image analysis was performed as described in (Bar *et al.,* 2016).

Based on the above methodology it was observed that long-term nilotinib administration induced a deterioration of endothelial function in the thoracic aorta in aged mice (Figure 1, Ach-induced vascular response: -12.62(2.58)% vs -4.83(1.66)%, for nilotinib-treated group vs untreated group), confirming the negative effect of chronic TKI therapy on vascular endothelial function, worsening the pre-existing age-related endothelial function impairment. After separate administration of MNA or vitamin K (K2-MK7), the impaired endothelial function induced by TKI administration returned at most to the level of age-related endothelial function impairment. In contrast, administration of the combination of MNA and vitamin K (K2- MK7) unexpectedly reversed the impaired endothelial function to the level observed in young mice. Indeed, after administration of the combination of compounds, Ach-induced endothelial response reached the level characteristic of healthy young individuals (7.50(0.63)% vs 7.53(0.54)% for the thoracic aorta, the values for the nilotinib+MNA+K2-MK7-treated group and for the young control group, respectively). Parallel measurements to assess the endothelium-independent response of the thoracic aorta to sodium nitroprusside (SNP) administration confirmedthat impaired vascular function wasrelated to endothelial dysfunction and not to impaired vascular muscle response (Figure 2).

### Conclusions:

The conducted studies show that the used combination of MNA and vitamin K unexpectedly not only completely prevents the negative effects of TKI therapy on vascular endothelial function, in elderly mice with pre-existing endothelial dysfunction, but also restores the state of impaired endothelial function to a state of completely normal endothelial function, an effect that is not induced by any of the agents administered alone.

The described invention therefore provides an unexpectedly beneficial, synergistically acting combination of vasoprotective compounds, the administration of which can be used as a supportive therapy to anticancer therapies characterized by side effects associated with adverse effects of the applied therapy on endothelial function resulting in cardiovascular complications of the anticancer therapy.

### Literature:

Bar, A. et al. (2016) 'Retrospectively gated MRI for in vivo assessment of endothelium-dependent vasodilatation and endothelial permeability in murine models of endothelial dysfunction', NMR in Biomedicine, 29(8), pp. 1088-1097. doi: 10.1002/nbm.3567.

Bar, A. et al. (2017) 'Functional and biochemical endothelial profiling in vivo in a murine model of endothelial dysfunction; comparison of effects of 1-methylnicotinamide and angiotensin-converting enzyme inhibitor', Frontiers in Pharmacology, 8(APR), pp. 1-13. doi: 10.3389/fphar.2017.00183.

Bar, A. et al. (2019) 'Vitamin K2-MK-7 improves nitric oxide-dependent endothelial function in ApoE/LDLR-/- mice', Vascular Pharmacology. Elsevier, 122-123(August), p. 106581. doi: 10.1016/j.vph.2019.106581.

Chlopicki, S. et al. (2007) '1-Methylnicotinamide (MNA), a primary metabolite of nicotinamide, exerts anti-thrombotic activity mediated by a cyclooxygenase-2/prostacyclin pathway', British Journal of Pharmacology. Wiley-Blackwell, 152(2), p. 230. doi: 10.1038/SJ.BJP.0707383.

Cortes, J. E. et al. (2018) 'Ponatinib efficacy and safety in Philadelphia chromosome-positive leukemia: final 5-year results of the phase 2 PACE trial', Blood. American Society of Hematology, 132(4), pp. 393-404. doi: 10.1182/BLOOD-2016-09-739086.

Douxfils, J. et al. (2016) 'Association Between BCR-ABL Tyrosine Kinase Inhibitors for Chronic Myeloid Leukemia and Cardiovascular Events, Major Molecular Response, and Overall Survival: A Systematic Review and Meta-analysis', JAMA Oncology. American Medical Association, 2(5), pp. 625-632. doi: 10.1001/JAMAONCOL.2015.5932.

Gover-Proaktor, A. et al. (2017) 'Ponatinib reduces viability, migration, and functionality of human endothelial cells', Leukemia and Lymphoma. Taylor and Francis Ltd, 58(6), pp. 1455-1467. doi: 10.1080/10428194.2016.1239258.

Gover-Proaktor, A. et al. (2019) 'Bosutinib, dasatinib, imatinib, nilotinib, and ponatinib differentially affect the vascular molecular pathways and functionality of human endothelial cells', Leukemia & Lymphoma. Taylor & Francis, 60(1), pp. 189-199. doi: 10.1080/10428194.2018.1466294. Hadzijusufovic, E. et al. (2017) 'Nilotinib-induced vasculopathy: identification of vascular endothelial cells as a primary target site', Leukemia. Europe PMC Funders, 31(11), p. 2388. doi: 10.1038/LEU.2017.245.

Haguet, H. et al. (2020) 'The Risk of Arterial Thrombosis in Patients With Chronic Myeloid Leukemia Treated With Second and Third Generation BCR-ABL Tyrosine Kinase Inhibitors May Be Explained by Their Impact on Endothelial Cells: An In-Vitro Study', Frontiers in Pharmacology. Frontiers Media S.A., 11. doi: 10.3389/FPHAR.2020.01007/FULL.

Herrmann, J. et al. (2016) 'Vascular Toxicities of Cancer Therapies: The Old and the New--An Evolving Avenue.', Circulation. NIH Public Access, 133(13), pp. 1272-89. doi: 10.1161/CIRCULATIONAHA.115.018347.

Hochhaus, A. et al. (2016) 'Long-term benefits and risks of frontline nilotinib vs imatinib for chronic myeloid leukemia in chronic phase: 5-year update of the randomized ENESTnd trial', Leukemia. Nature Publishing Group, 30(5), pp. 1044-1054. doi: 10.1038/LEU.2016.5.

lurlo, A. et al. (2023) 'Cardiovascular Adverse Events of Tyrosine Kinase Inhibitors in Chronic Myeloid Leukemia: Clinical Relevance, Impact on Outcome, Preventive Measures and Treatment Strategies', Current Treatment Options in Oncology. Springer, 24(12), pp. 1720-1738. doi: 10.1007/S11864-023-01149-1/FIGURES/3.

Kantarjian, H. M. et al. (2021) 'Long-term outcomes with frontline nilotinib versus imatinib in newly diagnosed chronic myeloid leukemia in chronic phase: ENESTnd 10-year analysis', Leukemia. Springer Nature, 35(2), pp. 440-453. doi: 10.1038/S41375-020-01111-2.

Kieronska et al., (2024) 'Phylloquinone improves endothelial function and inhibits cellular senescence and vascular inflammation' Geroscience 2024 https://doi.org/10.1007/s11357-024- 01225-w (in press).

Lin, S. et al. (2023) 'Qilong capsule alleviates ponatinib-induced ischemic stroke in a zebrafish model by regulating coagulation, inflammation and apoptosis', Journal of Ethnopharmacology. Elsevier, 314, p. 116397. doi: 10.1016/J.JEP.2023.116397.

Loren, C. P. et al. (2015) 'The BCR-ABL inhibitor ponatinib inhibits platelet immunoreceptor tyrosine-based activation motif (ITAM) signaling, platelet activation and aggregate formation under shear.', Thrombosis research. NIH Public Access, 135(1), pp. 155-60. doi: 10.1016/j.thromres.2014.11.009.

Mateuszuk, L. et al. (2016) 'Antiatherosclerotic Effects of 1-Methylnicotinamide in Apolipoprotein E/Low- Density Lipoprotein Receptor-Deficient Mice: A Comparison with Nicotinic Acid', The Journal of Pharmacology and Experimental Therapeutics. American Society for Pharmacology and Experimental Therapeutics, 356(2), p. 514. doi: 10.1124/JPET.115.228643.

Schmaier, A. H. et al. (2016) 'Ponatinib and Cardiovascular Complications', Blood, 128(22).

## Claims

1. A combination containing MNA and vitamin K for use in the treatment or prevention of vascular endothelial dysfunction.

2. The combination for use of claim 1, **characterized in that** the vascular endothelial dysfunction is induced by administration of a tyrosine kinase inhibitor.

3. A pharmaceutical composition comprising a substance selected from MNA and vitamin K for use in the treatment or prevention of vascular endothelial dysfunction induced by administration of a tyrosine kinase inhibitor.

4. The pharmaceutical composition for use of claim 3, **characterized in that** it contains MNA and vitamin K, preferably vitamin K2.
